# EUROPEAN PATENT SPECIFICATION

(11) **EP 0 555 618 B1**
(45) Date of publication and mention of the grant of the patent: **12.08.1998**
(21) Application number: 92830653.9
(22) Date of filing: 04.12.1992
(51) Int. Cl.: A23L 1/03, A61K 35/74

(54) **Dietetic and/or pharmaceutical compositions containing lyophilized lactic bacteria**
Lyophilisierte Bakterien enthaltende Diät- oder Arzneimittelnzusammenstellungen
Compositions diététiques ou pharmaceutiques comprenant des bactéries lactiques lyophilisées

(30) Priority: 10.02.1992 IT MI920256
(43) Date of publication of application: 18.08.1993
(73) Proprietor: Cavaliere Vesely, Renata Maria Anna, I-20123 Milano (IT); De Simone, Claudio, I-00040 Ardea (Roma) (IT)
(72) Inventor: Cavaliere Vesely, Renata Maria Anna, I-20123 Milano (IT); De Simone, Claudio, I-00040 Ardea (Roma) (IT)
(74) Representative: Righetti, Giuseppe

(56) References cited:
- EP-A- 0 228 861
- EP-A- 0 291 578
- US-A- 4 806 368
- US-A- 5 143 845
- DATABASE WPI Section Ch, Week 8916, Derwent Publications Ltd., London, GB; Class A96, AN 89-119459 & JP-A-1 066 124 (FREUND SANGYO) 13 March 1989
- PATENT ABSTRACTS OF JAPAN vol. 9, no. 309 (C-318)5 December 1985 & JP-A-60 149 527 (SEIKENKAI) 7 August 1985
- MICROECOLOGY AND THERAPY vol. 14 , 1984 , HERBORN-DILL pages 109 - 126 Y.KAWAI 'Effect of cellular extracts of streptococci on hyperlipidemia in rats,rabbits and humans'
- BIFIDOBACTERIA MICROFLORA vol. 3, no. 1 , 1984 pages 29 - 33 T.KAGEYAMA ET AL. 'The effect of bifidobacterium administration in patients with leukemia'
- BIFIDOBACTERIA MICROFLORA vol. 1, no. 1 , 1982 pages 3 - 24 T.MITSUOKA 'Recent trends in research on intestinal flora'
- MICROECOLOGY AND THERAPY vol. 16 , 1986 , HERBORN-DILL pages 271 - 272 D.MÜTING ET AL. 'Bifidobacterium bifidus administration in humans:a controlled clinical study in liver cirrhosis'
- MICROECOLOGY AND THERAPY vol. 15 , 1985 , HERBORN-DILL pages 271 - 280 N.SUEGARA ET AL. 'Hypolipidemic effect of streptococcus faecalis kawai in humans and mechanisms of serum lipid reduction'

## Description

The present invention relates to a dietetic and/or pharmaceutical composition containing lyophilized lactic bacteria which are qualitatively and quantitatively coordinated in an optimal manner for carrying out a re-balancing action on the intestinal flora, as well as a hypocholesterolemic and immunomodulating action. More specifically, the present invention relates to a dietetic and/or pharmaceutical composition capable of normalizing the functions of the intestinal flora and promoting the body welfare, said composition containing lyophilized lactic bacteria, an excipient and optionally a physiologically compatible drug.

In Bifidobacteria Microflora, Vol. 3(1), 29-33, 1984, the beneficial effect of administering Bifidobacterium to patients suffering from leukemia is described.

In FEMS Microbiology Reviews 46 (1987), 343-356, the therapeutical function of lactobacilli is disclosed, while Nobuo Suegara et al. (Microecology and Therapy, Vol. 15, 271-280 (1985)) state that oral administration of S. faecalis KAWAI greatly improves the lipid metabolism in human beings and animals.

From Microecology and Therapy, Vol. 14, 109-126 (1984) the effect of streptococcus cell extracts on hyperlipemia in rats, rabbits and human beings is known.

Other references describing the beneficial action of lactobacilli or other strains of activated bacilli are for example Bifidobacteria Microflora 1, 3-24, 1982 (Recent Trends in Research on Intestinal Flora), Microecology and Therapy 14, 267, 1984 (Intestinal Flora Associated Endotoxin), Microecology Therapy 16, 271-272, 1986 (Bifidobacterium bifidum Administration in Humans: a Controlled Clinical Study in Liver Cirrhosis), etc.

In IT-B-1022625 food and farmaceutical compositions are described which perform an activity stimulating the production of gamma-interferon and contain lactobacilli S. thermophilus and L. bulgaricus.

US-A-4806368 deals with the problem of enhancing the viability of Lactobacillus acidophilus and other bacteria in tablets and also in gastro-intestinal tract and discloses a dietary beneficial bacteria tablet comprising lyophilized live bacteria, apple fibers, a vitamin having antioxidant properties, an amino acid having reducing properties, an alkaline mineral salt, and means for stimulating bacterial growth. The bacteria are selected from Lactobacillus acidophilus, Bifidobacterium bifidum, Propionibacterium shermanii, Leuconostoc citrovorum, Streptococcus thermophilus, Lactobacillus bulgaricus, Streptococcus lactis, Streptococcus cremoris, Streptococcus durans, Streptococcus faecalis, Lactobacillus casei, Lactobacillus caucasicus, Lactobacillus lactis and Lactobacillus helveticus. In particular this document discloses some specific tablets wherein the bacteria are: Lactobacillus acidophilus (see Examples 1, 2, 3 and 4); Lactobacillus acidophilus and/or Bifidobacterium bifidum (see Example 1); Lactobacillus acidophilus, Propionibacterium shermanii and Leuconostoc citrovorum (see Example 5); and a combination of fifteen bacteria, namely Lactobacillus acidophilus, Bifidobacterium bifidum, Propionibacterium shermanii, Leuconostoc citrovorum, Streptococcus thermophilus, Lactobacillus bulgaricus, Lactobacillus casei, Lactobacillus caucasicus, Lactobacillus lactis, Lactobacillus helveticus, Streptococcus lactis, Streptococcus cremoris, Streptococcus diactilactis, Streptococcus durans and Streptococcus faecalis (see Example 6, wherein the amount of each of the fifteen bacteria is the same and the total concentration of bacteria is at most 68 x 10⁶ per 105 mg of bacteria).

DATABASE WPI, Section Ch, Week 8916, Derwent Publications; Class A96, AN 89-119459 & JP-A-1066124 (FREUND SANGYO) discloses granules containing useful bacteria, e.g. Bifidobacterium bifidum, B. longum, B. adolescentis, B. breve, B. infantis, Lactobacillus acidophilus, L. lactis, L. casei, L. bulgaricus and Streptococcus lactis, with a concentration of 1 x 10¹⁰ bacteria per gram. The granulation is carried out with a shellac solution, thus obtaining granules which are stable in the stomach, soluble in intestines and useful for the prevention of proliferation of harmful bacteria and maintenance of health.

It is an object of the present invention to provide an appropriate dietetic and/or pharmaceutical composition comprising lyophilized lactic bacteria qualitatively and quantitatively coordinated in an optimal manner for carrying out a re-balancing action on the intestinal flora, as well as a hypocholesterolemic and immunomodulating action, said composition containing, in addition to lyophilized lactic bacteria, also usual excipients, optionally together with some physiologically compatible drugs.

The essential features of the composition according to the present invention are defined in claim 1. Specific embodiments are defined in the dependent claims.

The dietetic and/or pharmaceutical composition according to the present invention contains lyophilized lactic bacteria which are capable of promoting a welfare state in mammals, for example antagonizing the onset of diarrhoea, constipation, hypercholesteremia, liver disorders, immunosuppressant, enteritis, endotoxin absorption and production of endogenous toxic substances. It has been particularly found that the new pharmaceutical composition object of the present invention is suitable for use in restoring the intestinal mucous membrane mass and modulating the epithelial cell kinetics and the enzyme potential of the intestine when they have been altered as a result of stress conditions. Other therapeutical suggestions for the composition of the invention comprise: gastroenteritis, colitis, constipation, diarrhoea, recolonization of the intestine after disorders due to foods, drugs, chemical or physical agents, after surgical interventions, chemotherapy or contagious deseases, hepatopathies resulting from toxic, metabolic or infectious causes, as an adjuvant for controlling hyperammonemia and endotoxinemia while liver insufficiency is in progress, in case of lack of humoral immunity and of mucosal and systemic cell-mediated immunity associated with secondary immunological deficiency diseases, hyperlipoproteinemia and hypercholesteremia, as an adjuvant in diabetes therapy.

Moreover, the dietetic and/or pharmaceutical composition according to the present invention is capable of normalizing the functions of the intestinal flora and promoting the body welfare, in that it promotes the synthesis of vitamins and proteins, digestive, enzymatic and absorption processes, prevents colonization of pathogenic germs, stimulates the immune response and counteract arising of diarrhoea, constipation, enteritis, production of endogenous substances, absorption of endotoxins. This composition finds thus application in preventing and treating liver disorders, hypercholesteremia and immunosuppressant.

The new lyophilized bacteria-containing composition of the invention can be prepared by treating bacteria with appropriate technical expedients enabling the bacterium vitality to be recovered when they reach the digestive system. As a result, the administration of the composition in question modifies the intestinal microflora, affects the expression of the membrane enzymes of microvilli and promotes the integrity of intestinal epithelial cells and related structures. The re-balancing action performed by the composition of the invention in the intestine is followed by a reduction in the production of substances undesired by the proteolytic flora and a decreased absorption of endotoxins in the entire bodily system and a variation in the amount of cytokines produced by the intestinal wall and present in faeces. The synergism of these actions induces an improvement in the clinical symptomatology and the laboratory parameters in subjects suffering from hepatopathies and trouble of the intestinal motility associated with dyspeptic-enterocolitis syndromes.

Bacteria present in the composition object of the invention survive in the intestine, colonize it in a short time and stimulate the local and systemic immune apparatus. Studies about immunopharmacology have highlighted a development or normalization in the production of cytokines, among which interferons, interleukins and tumor necrosi factor (TNF) after ingestion of the compositions. By reactivating the humoral and cell-mediated immune system, the compositions can be used for the prophylaxis and therapy of immunological alterations associated with secondary immunological deficiency desease or stress situations.

Since some bacterial strains present in the composition are capable of absorbing cholesterol, they also have a hypocholesterolemic and anti-atherosclerosis action. Another mechanism through which the composition lower the cholesterol levels in the blood is the stimulation of cholesterol excretion in faeces.

Lyophilized bacteria, i.e. active ingredients, that are inserted in the composition of the invention are Bifidobacterium longum, Bifidobacterium bifidum, Bifidobacterium infantis, L. acidophilus, L. casei, L. delbrueckii sub-species bulgaricus, L. plantarum, S. thermophilus and S. faecium.

As further components, the composition of the invention contains usual excipients currently employed for preparing pharmaceutical compositions.

The composition of the invention can be made in the usual pharmaceutical forms known in literature, such as for example tablets, coated tablets, capsules, packets, solutions, suspensions, emulsions, suppositories, pellets, syrups, vaginal suppositories, ointments, creams, and is prepared in the usual manner by mixing the active ingredients with excipients, optionally adding adjuvants and/or dispersing agents; should water be used as the diluent, also other organic solvents can be used in the form of adjuvants. Adjuvants can be for example: water, non-toxic organic solvents such as paraffines, vegetable oils (peanut oil or sesame oil) alcohols (ethanol, glycerine, for example), glycols (propylenglycol, polyethylenglycol), solid carriers such as for example natural mineral flours (kaolin, talc), synthetic mineral flours (silicates for example), sugar (cane sugar for example), emulsifiers (alkylsulfonates or arylsulfonates and the like), dispersers (lignin, methylcellulose, starch and polyvinylpyrrolidone, for example) and lubricants (magnesium stearate, talc, stearic acid, sodium laurylsolfonate, for example).

The administration takes place in the usual manner, preferably by oral route. In this case pharmaceutical forms adapted to this end can contain, in addition to usual excipients such as lactulose, dextrose, lactose, also additives such as sodium citrate, calcium carbonate, calcium dihydrogen phosphate, together with several additional substances such as starch, gelatin and the like. In case of liquid forms compatible colouring agents or flavoring substances may be added.

As an optional component, the composition of the invention may contain a drug compatible with the bacteria used, capable of strengthening the activity of the active ingredients present therein. Among these drugs, the following can be mentioned: anticonvulsant, anticholinergic, antihistaminic, adrenergic, sedative, antiinflammatory, antipyretic, antiseptic, analgesic, antirheumatic, diuretic, antipsychotic, antibacterial, hepatoprotector, antilipemic drugs and analeptic drugs.

Also active ingredients obtained from the bacterial walls or other bacterium cell components having a biological pro-host activity may be added.

For preparing the composition, the individual microorganisms in the lyophilized form are mixed in appropriate proportions and the mixture is then added with the excipients and optionally the drug.

Both the individual strains and the final mixture have been submitted to microbiological tests for the purpose of evaluating both the number of microorganisms and possible pollutants. For counting of the bacteria forming the mixture, the following media have been employed:
- Streptococci: M17 Agar - incubation over 48 hours
- Lactobacilli and bifidobacteria: MRS Agar - incubation in anaerobiosis at 37°C over 72 hours

For counting of possible pollutants the following media have been employed:
- Coliiformes: deoxycholate agar - inoculation at 32°C over 24 hours
- Enterococci: KF agar + TTC incubation at 37°C over 48 hours
- Non-lactic flora: gel agar - incubation at 32°C over 72 hours

Microbiological tests on the mixtures have given the following average results:
- Coliiformes: < 100
- Enterococci: absent/g
- Non-lactic flora: absent/g

From the above results it is possible to conclude that, for the entirety of the microorganisms being part of the compositions, high charges have been achieved by virtue of the optimal conditions both of growth, and of concentration and drying up.

### Pharmacological Part

Studies carried out on healthy volunteers or volunteers suffering from some of the above mentioned pathologies have proved the efficiency of the compositions in question.

Reproduced hereinafter are some of the parameters that have been measured according to the good usual medical procedure carried out in laboratory, that is: cholesterol, GOT, T CD4+ lymphocytes/CD8+ lymphocytes ratio, GPT, gamma GT and NK (natural killer) activity.

| CHOLESTEROL (mg/dl) | | | | |
|---|---|---|---|---|
| | time 0 | time 1 | time 2 | time 3 |
| average | 190,6 | 179,0 | 174,4 | 184,2 |
| Maximum | 285,3 | 264,8 | 247,5 | 267,4 |
| Minimum | 108,3 | 118,1 | 108,6 | 113,6 |
| SD | 59,9 | 44,7 | 44,0 | 54,3 |

| GOT (IU/ml) | | | | |
|---|---|---|---|---|
| | time 0 | time 1 | time 2 | time 3 |
| average | 29.8 | 25,8 | 26,0 | 23,3 |
| Maximum | 97,3 | 88,1 | 81,5 | 59,1 |
| Minimum | 13,2 | 6,8 | 10,8 | 10,8 |
| SD | 23,1 | 22,0 | 19,1 | 13,7 |

| CD4 TO CD8 RATIO | | | | |
|---|---|---|---|---|
| | time 0 | time 1 | time 2 | time 3 |
| average | 1,45 | 1,75 | 1,64 | 1,27 |
| Maximum | 2,71 | 4,10 | 4,32 | 2,76 |
| Minimum | 0,96 | 1,05 | 0,97 | 0,58 |
| SD | 0,57 | 0,87 | 0,95 | 0,60 |

**GPT (IU/ml)**

| | time 0 | time 1 | time 2 | time 3 |
|---|---|---|---|---|
| average | 31,9 | 31,2 | 26,6 | 24,5 |
| Maximum | 131,6 | 132,6 | 99,1 | 91,0 |
| Minimum | 10,1 | 7,1 | 7,5 | 6,7 |
| SD | 34,5 | 35,4 | 26,3 | 24,1 |

| gamma GT (IU/ml) | | | | |
|---|---|---|---|---|
| | time 0 | time 1 | time 2 | time 3 |
| average | 41,2 | 40,3 | 37,5 | 38,5 |
| Maximum | 127,0 | 137,8 | 129,1 | 125,6 |
| Minimum | 12,3 | 12,5 | 9,2 | 9,7 |
| SD | 34,3 | 37,1 | 35,4 | 34,3 |

| NK Activity (12.5:1) | | | | |
|---|---|---|---|---|
| | time 0 | time 1 | time 2 | time 3 |
| average | 44,0 | 67,5 | 49,9 | 41,0 |
| Maximum | 62,0 | 97,0 | 62,0 | 56,0 |
| Minimum | 24,0 | 47,0 | 25,0 | 14,0 |
| SD | 12,2 | 16,9 | 11,4 | 14,1 |

| NK Activity (25:1) | | | | |
|---|---|---|---|---|
| | time 0 | time 1 | time 2 | time 3 |
| average | 56,4 | 80,5 | 54,6 | 48,1 |
| Maximum | 66,0 | 100,0 | 69,0 | 63,0 |
| Minimum | 42,0 | 51,0 | 37,0 | 23,0 |
| SD | 9,8 | 13,3 | 9,5 | 12,0 |

| NK Activity (50:1) | | | | |
|---|---|---|---|---|
| | time 0 | time 1 | time 2 | time 3 |
| average | 60,8 | 87,8 | 59,1 | 51,8 |
| Maximum | 74,0 | 100,0 | 65,0 | 63,0 |
| Minimum | 48,0 | 69,0 | 42,0 | 30,0 |
| SD | 7,5 | 10,5 | 6,9 | 11,1 |

### EXAMPLE

Strains used in the following formulation, given by way of example only, are as follows:
- thermophile Streptococci consist of a mixture of two strains from a yogurt culture and a starter used for preparation of cheeses;
- Lactobacillus bulgaricus is represented by a strain isolated from a yogurt culture;
- Lactobacillus acidophilus is present in a mixture consisting of two strains of human origin isolated from a special yogurt;
- Bifidobacteria come from the intestinal flora of newborn babies;
- Lactobacillus casei has been isolated from a culture employed in the production of cheeses, and
- Lactobacillus plantarum has been isolated from vegetables in progress of fermentation.

In order to obviate problems due to possible phage attacks, these strains can obviously be replaced by other cultures having the same features and origins, but provided with a different phage sensitiveness.

### Culture Preparation

The individual strains maintained in a lyophilized and frozen form have been grown in synthetic media specific for each species. The fundamental component of the culture medium is permeate obtained by ultrafiltration of serum or milk, to which minima amounts of biological activators are added depending on the species. After sterilization, the culture medium is inoculated with a strain per species or 1 to 3 strains belonging to the same genotype. Cultures have been incubated upon determination of optimal parameters for each strain: temperature, time, pH values and stirring.

Industrial cultures have been concentrated by centrifugation, and lyophilization has been then carried out according to standard methodologies.

After lyophilisation the cell mass has been pulverized under sterile conditions. The individual cultures submitted to chemical and microbiological tests have been maintained at 5°C in hermetic vessels.

### Preparation of the individual species

### 1) Streptococcus salivarius sub-species thermophilus

### - Mother

The mother has been prepared by inoculating the strain in a medium consisting of 5% of permeate + 1% of yeast extract and incubated at 44°C over 3 hours.

### - Medium

| | |
|---|---|
| Permeate | 5% |
| Yeast extract | 1% |

### - Fermentation parameters

| | |
|---|---|
| Fermenter: | 72 l Applikon |
| Percent of inoculation: | 1% |
| Incubation temperature | 44°C |
| Stirring speed | 160 rpm |
| Neutralization set point | pH = 6.00 |
| Neutralizing substance type: | ammonium hydrate (sol to 10%) |
| Fermentation time: | 3h 30m |
| Final cooling: | 24°C |

### - Concentration parameters

| | |
|---|---|
| Centrifuge type: | Westfalia SA1 |
| Centrifugation temperature: | 24°C |
| flow rate: | 24 l/h |

(The concentrate has been then centrifuged again using a laboratory centrifuge at 6000 rpm over 20 minutes).

### - Lyophilization

| | |
|---|---|
| Lyophilizer | Edwards MINI - FAST 3400 |
| Lyophilization protector: | a solution of lactose |

Results: the number of microorganisms during the different steps of the process are reproduced in the following Table:

| Steps | U.F.C./g |
|---|---|
| End of fermentation | 2.4E9 |
| Concentrate | 1.6E11 |
| Lyophilized | 7.4E11 |
| U.F.C. = Colony-forming units E9 = one thousand millions E11 = one hundred thousand millions | |

No particular problems have been, found in the preparation of this microorganism. Therefore the cell loss during the different steps of the process could be greatly limited and a high bacterial charge could be achieved in the lyophilized product.

### 2) Lactobacillus plantarum

### - Mother

Prepared in MRS culture medium and incubated at 33°C over 5 hours.

### - Medium

| | |
|---|---|
| Permeate | 5% |
| Yeast extract | 1% |
| Glucose | 2.5% |

### - Fermentation parameters

| | |
|---|---|
| Fermenter: | Applikon |
| Percent of inoculation: | 1% |
| Incubation temperature | 33°C |
| Stirring speed | 110 rpm |
| Neutralization set point | pH = 6.00 |
| Neutralizing substance type: | ammonium hydrate (sol to 10%) |
| Fermentation time: | 15 h |

Cell inactivation after fermentation by pasteurization at 80°C over 15 minutes.

### - Concentration parameters

| | |
|---|---|
| Centrifuge type: | Westfalia SA1 |
| Centrifugation temperature: | 60°C |
| flow rate: | 40 l/h |

### - Lyophilization

| | |
|---|---|
| Lyophilizer | Edwards MINI - FAST 3400 |
| Lyophilization protector: | a solution of lactose |

The number of microorganisms during the different process steps is reproduced in the following Table:

| Steps | U.F.C./g | Count/g in Thoma |
|---|---|---|
| End of fermentation | 9.2E8 | - |
| Lyophilized | - | 1.0E11 |
| U.F.C. = Colony-forming units E8 = one hundred millions E11 = one hundred thousand millions. | | |

### 3) Lactobacillus casei

### - Mother

Prepared in MRS culture medium and incubated at 37°C over 8 hours and 30 minutes

### - Medium

| | |
|---|---|
| Permeate | 5% |
| Yeast extract | 1% |
| Glucose | 1% |

### - Fermentation parameters

| | |
|---|---|
| Fermenter: | Applikon |
| Inoculation percent: | 1% |
| Incubation temperature | 37°C |
| Stirring speed | 110 rpm |
| Neutralization set point | pH = 5.40 |
| Neutralizing substance type: | ammonium hydrate (sol to 10%) |
| Fermentation time: | 15 h |

### - Concentration parameters

| | |
|---|---|
| Centrifuge type: | Westfalia SA1 |
| Centrifugation temperature: | 60°C |
| Flow rate: | 46 l/h |

### - Lyophilization

| | |
|---|---|
| Lyophilizer | Edwards MINI - FAST 3400 |
| Lyophilization protector: | a lactose solut. |

Results: the number of microorganisms during the different process steps is reproduced in the following Table:

| Steps | U.F.C./g | Count/g in Thoma |
|---|---|---|
| End of fermentation | 1.0E9 | - |
| Lyophilized | - | 1.0E11 |
| U.F.C. = Colony-forming units E8 = one thousand millions E11 = one hundred thousand millions. | | |

### 4) Mixture of bifidobacteria (Bifidobacterium infantis -Bifidobacterium longum - Bifidobacterium bifidum)

### - Mother

The mother has been prepared by inoculating the strains in a medium consisting of 10% of powdered skimmed milk + 0.5% of glucose + 1% of yeast extract and incubated at 38°C over 15 hours.

### - Medium

| | |
|---|---|
| Permeate | 4% |
| Yeast extract | 1% |
| Bacto Soytone | 0.25% |
| Glucose | 0.5% |

### - Fermentation parameters

| | |
|---|---|
| Fermenter: | Applikon |
| Inoculation percent: | 2% |
| Incubation temperature | 38°C |
| Stirring speed | 110 rpm |
| Neutralization set point | pH = 6.00 |
| Neutralizing substance type: | ammonium hydrate (sol to 10%) |
| Fermentation time: | 15 h |
| Cooling at the end of fermentation: | 24°C |

### - Concentration parameters

| | |
|---|---|
| Centrifuge type: | Westfalia SA1 |
| Centrifugation temperature: | 24°C |
| Flow rate: | 42 l/h |

(The obtained concentrate has been then centrifuged again with a laboratory centrifuge at 6000 rpm over 20 minutes).

### - Lyophilization

| | |
|---|---|
| Lyophilizer | Edwards MINI - FAST 3400 |

Lyophilization protector: a solution of powdered slimmed milk + yeast extract + lactose + sodium maleate has been prepared.

Results: the number of microorganisms during the different process steps is reproduced in the following Table:

| Steps | U.F.C./g |
|---|---|
| End of fermentation | 1.7E9 |
| Concentrate | 7.0E10 |
| Lyophilized | 3.8E11 |
| U.F.C. = Colony-forming units E9 = one thousand millions E10 = ten thousand millions E11 = one hundred thousand millions. | |

In this case too, in which bacteria are considered of "hard" growing, no particular problems have been found during the different preparation steps and the number of microorganisms is high both in the fermentation and on the lyophilized.

### 5) Lactobacillus acidophilus

### - Mother

Prepared in a medium consisting of 5% of permeate + 1% of yeast extract + 1% of glucose + 1% of Tween (Registered Trademark) 80 and incubated at 37°C over 15 hours.

### - Medium

| | |
|---|---|
| Permeate | 5% |
| Yeast extract | 1% |
| Glucose | 1% |
| Tween (Registered Trademark) 80 | 0.1% |

### - Fermentation parameters

| | |
|---|---|
| Fermenter: | Applikon |
| Inoculation percent: | 1% |
| Incubation temperature | 37°C |
| Stirring speed | 110 rpm |
| Neutralization set point | pH = 6.00 |
| Neutralizing substance type: | ammonium hydrate (sol to 10%) |
| Fermentation time: | 15 h |
| Cooling at the end of fermentation: | 24°C |

### - Concentration parameters

| | |
|---|---|
| Centrifuge type: | Westfalia SA1 |
| Centrifugation temperature: | 24°C |
| Flow rate: | 42 l/h |

(The obtained concentrate has been then centrifuged again with a laboratory centrifuge at 6000 rpm over 20 minutes).

### - Lyophilization

| | |
|---|---|
| Lyophilizer | Edwards MINI - FAST 3400 |

Lyophilization protector: a solution of lactose and anhydrous mixture consisting of powdered skimmed milk + yeast extract + lactose + sodium glutamate + Tween (Registered Trademark) 80.

Results: the number of microorganisms during the different process steps is reproduced in the following Table:

| Steps | U.F.C./g |
|---|---|
| End of fermentation | 2.9E8 |
| Concentrate | 2.3E10 |
| Lyophilized | 2.0E10 |
| U.F.C. = Colony-forming units E8 = one hundred millions E10 = ten thousand millions | |

### 6) Lactobacillus delbrueckii sub-species bulgaricus

### - Mother

Prepared in a medium consisting of 5% of permeate + 1% of yeast extract + 1% of beef extract + 1% of glucose + 0.1% of Tween (Registered Trademark) 80 and incubated at 44°C over 4 hours and 30 minutes.

### - Medium

| | |
|---|---|
| Permeate | 5% |
| Yeast extract | 1% |
| Beef extract | 1% |
| Glucose | 1% |
| Tween (Registered Trademark) 80 | 0.1% |

### - Fermentation parameters

| | |
|---|---|
| Fermenter: | Applikon |
| Inoculation percent: | 1% |
| Incubation temperature | 44°C |
| Stirring speed | 110 rpm |
| Neutralization set point | pH = 5.60 |
| Neutralizing substance type: | ammonium hydrate (sol to 10%) |
| Fermentation time: | 7 h |
| Cooling at the end of fermentation: | 24°C |

### - Concentration parameters

Installation: pilot unit for Hydro Air Research microfiltration with two serial ceramic diaphragms each having a 0.2 m² filtrating surface.

| | |
|---|---|
| Microfiltration temperature | 30°C |
| Operating conditions | recirculation flow rate 4000 l/h input pressure 2.7 bar output pressure 1.2 bar average flow of the permeate 30 l/h x m² |

(The obtained concentrate has been then centrifuged again with a laboratory centrifuge at 6000 rpm over 20 minutes).

### - Lyophilization

| | |
|---|---|
| Lyophilizer | Edwards MINI - FAST 3400 |

Lyophilization protector: a solution of lactose and anhydrous mixture consisting of powdered skimmed milk + yeast extract + lactose + sodium glutamate + Tween (Registered Trademark) 80.

Results: the number of microorganisms during the different process steps is reproduced in the following Table:

| Steps | U.F.C./g |
|---|---|
| End of fermentation | 2.9E9 |
| Concentrate | 2.4E10 |
| Lyophilized | 3.5E9 |
| U.F.C. = Colony-forming units E9 = one thousand millions E10 = ten thousand millions | |

## Claims

1. A dietetic and/or pharmaceutical compositions capable of normalizing the functions of the intestinal flora and of promoting the body welfare in human beings and animals, said composition consisting essentially of:
- a mixture of lyophilized bacteria,
- up to 10% by weight of a pharmacologically acceptable excipient, and
- optionally up to 20% by weight of a physiologically compatible drug, said mixture of lyophilized bacteria consisting of:
- 10 to 40% by weight of S. thermophilus, in a concentration of 7.4x10¹¹ bacteria per gram,
- 1 to 15% by weight of L. casei, in a concentration of 1.0x10¹¹ bacteria per gram,
- 1 to 20% by weight of L. plantarum, in a concentration of 1.0x10¹¹ bacteria per gram,
- 10 to 40% by weight of a mixture of Bifidobacterium longum, Bifidobacterium bifidum and Bifidobacterium infantis, in a concentration of 3.8x10¹¹ bacteria per gram,
- 1 to 15% by weight of L. acidophilus, in a concentration of 2.0x10¹¹ bacteria per gram,
- 1 to 20% by weight of L. bulgaricus, in a concentration of 3.5x10⁹ bacteria per gram, and
- 1 to 20% by weight of S. faecium, in a concentration of 1x10⁹ to 5x10¹² bacteria per gram,
and said percentages of excipient and drug being based on the total weight of bacteria.

2. The composition according to claim 1, characterized in that the drugs are selected from anticonvulsant, anticholinergic, antihistaminic, adrenergic, sedative, antiinflammatory, antipyretic, antiseptic, analgesic, antirheumatic, diuretic, antipsychotic, antibacterial, hepatoprotector, antilipemic and analeptic drugs.

3. A composition according to claim 1 and/or 2, characterized in that it is in the form of tablets, coated tablets, capsules, packets, solutions, emulsions, suspensions, pellets, suppositories, syrups, vaginal suppositories, ointments or creams.

## Patentansprüche

1. Diätetische und/oder pharmazeutische Zusammensetzungen, die fähig sind, die Funktionen der Darmflora zu normalisieren und das Wohlbefinden des Organismus im Menschen und in den Tieren zu fördern, wobei die Zusammenstellung im wesentlichen besteht aus:
- einer Mischung von lyophilisierten Bakterien,
- bis zu 10 Gew.-% eines pharmakologisch annehmbaren Bindemittels, und
- eventuell bis zu 20 Gew.-% eines physiologisch verträglichen Arzneimittels,
wobei die Mischung von lyophilisierten Bakterien umfaßt:
- von 10 bis 40 Gew.-% von S. Thermophilus, in einer Konzentration von 7,4x10¹¹ Bakterien pro Gramm,
- von 1 bis 15 Gew.-% von L. Casei, in einer Konzentration von 1,0x10¹¹ Bakterien pro Gramm,
- von 1 bis 20 Gew.-% von L. Plantarum, in einer Konzentration von 1,0x10¹¹ Bakterien pro Gramm,
- von 10 bis 40 Gew.-% einer Mischung von Bifidobacterium longum, Bifidobacterium bifidum und Bifidobacterium infantis, in einer Konzentration von 3,8x10¹¹ Bakterien pro Gramm,
- von 1 bis 15 Gew.-% von L. Acidophilus, in einer Konzentration von 2,0x10¹¹ Bakterien pro Gramm,
- von 1 bis 20 Gew.-% von L. Bulgaricus, in einer Konzentration von 3,5x10¹¹ Bakterien pro Gramm, und
- von 1 bis 20 Gew.-% von S. Faecium, in einer Konzentration von 1x10⁹ bis 5x10¹² Bakterien pro Gramm, und wobei diese Anteile von Bindemitteln und Arzneimitteln auf dem Gesamtgewicht der Bakterien beruhen.

2. Zusammensetzung nach Anspruch 1, dadurch gekennzeichnet, daß die Arzneimittel und den antispasmodischen, anticholinergischen, antiistaminischen, adrenergischen, sedativen, antientzündlichen, antipyretischen, antiseptischen, analgetischen, antireumathischen, diuretischen, antipsychotischen, antibakteriellen, leberschützenden, antilipämischen und analeptischen Arzeimitteln ausgewählt sind.

3. Zusammensetzung nach Anspruch 1 und/oder 2, dadurch gekennzeichnet, daß sie in der Form von Tabletten, überzogenen Tabletten, Kapseln, Tütchen, Lösungen, Emulsionen, Suspensionen, Granulaten, Zäpfchen, Sirup, Vaginalzäpfchen, Salben oder Cremen vorliegt.

## Revendications

1. Compositions diététiques ou pharmaceutiques en mesure de normaliser les fonctions de la flore intestinale et de favoriser le bien-être de l'organisme dans les humains et les animaux, ladite composition comportant essentiellement:
- un mélange de bactéries lyophilisées,
- jusqu'à 10% en pois d'un excipient acceptable du point de vue pharmacologique, et
- éventuellement jusqu'à 20% en poids d'un médicament physiologiquement compatible,
ledit mélange de bactéries lyophilisées se composant de:
- 10 à 40% en poids de *S. thermophilus*, en une concentration de 7,4x10¹¹ bactéries par gramme,
- 1 à 15% en poids de *L. casei*, en une concentration de 1,0x10¹¹ bactéries par gramme,
- 1 à 20% en poids de *L. plantarum*, en une concentration de 1,0x10¹¹ bactéries par gramme,
- 10 à 40% en poids d'un mélange de *Bifidobacterium longum*, *Bifidobacterium bifidum* et *Bifidobacterium infantis*, en une concentration de 3,8x10¹¹ bactéries par gramme,
- 1 à 15% en poids de *L. acidophilus*, en une concentration de 2,0x10¹¹ bactéries par gramme,
- 1 à 20% en poids de *L. bulgaricus*, en une concentration de 3,5x10⁹ bactéries par gramme, et
- 1 à 20% en poids de *S. faecium*, en une concentration de 1x10⁹ à 5x10¹² bactéries par gramme,
et lesdits pourcentages d'excipient et de médicament se basant sur le poids total des bactéries.

2. Composition selon la revendication 1, caractérisée en ce que les médicaments sont choisis parmi les médicaments anticonvulsifs, anticholinergiques, antihistaminiques, adrénergiques, sédatifs, anti-inflammatoires, antipyrétiques, antiseptiques, analgésiques, antirhumatismaux, diurétiques, antipsychotiques, antibactériens, hépato-protecteurs, antilipémie et analeptiques.

3. Composition selon la revendication 1 et/ou 2, caractérisée en ce qu'elle se présente sous forme de comprimés, comprimés enrobés, capsules, sachets, solutions émulsions, suspensions, granulés, suppositoires, sirops, ovules vaginaux, onguents ou crèmes.
